(19) Europäisches Patentamt / European Patent Office / Office européen des brevets

(11) **EP 2 887 379 A1**

(12) **EUROPEAN PATENT APPLICATION**

(43) Date of publication:
**24.06.2015 Bulletin 2015/26**

(51) Int Cl.:
**H01J 27/24** *(2006.01)*   *H01J 33/00* *(2006.01)*
**A61N 5/10** *(2006.01)*

(21) Application number: **13380060.7**

(22) Date of filing: **23.12.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **Proton Laser Applications, S.L.**
  **37007 Salamanca (ES)**
• **Consejo Superior De Investigacíon Científicas
  (CSIC)**
  **28006 Madrid (ES)**
• **Universidad Politecnia de Valencia
  46022 Valencia (ES)**
• **Centro de Laseres Pulsados
  Ultracortos Ultraintensos
  37008 Salamanca (ES)**

(72) Inventors:
• **Benlloch Baviera, José Maria
  46022 Valencia (ES)**
• **Roso Franco, Luis
  37008 Salamanca (ES)**
• **Voces Sánchez, Felipe
  37007 Salamanca (ES)**
• **Seimetz, Michael
  46022 Valencia (ES)**

(74) Representative: **Grünecker Patent- und
Rechtsanwälte
PartG mbB
Leopoldstraße 4
80802 München (DE)**

(54) **Intra-operatory carbon ion radiation therapy system**

(57)   Intra-operatory ion therapy system for irradiating a patient with laser-accelerated ions, preferably carbon ions, henceforth referred to as "Ion therapy system", comprising a laser radiation source adapted to emit laser radiation, a housing, being arranged and connected to the laser radiation source, such that laser radiation emitted from the laser radiation source can be coupled into the housing, a laser target onto which the laser radiation emitted from the laser radiation source can be focussed and from which surface an ion beam can be generated, a window portion in the housing adapted to let an incident ion beam pass the window portion to the outside of the housing at minimum energy loss and adapted to seal the housing, a vacuum system for generating lower pressure in the inner space of the housing, a focussing system for focussing the laser radiation on the laser target, holding means for holding the laser target and a control device for controlling the focussing system and the holding means and for adjusting the propagation direction of the ion beam such that the ion beam impinges on an external target, wherein the laser radiation source is adapted to generate laser pulses with a duration between $10^{-18}$s and $10^{-12}$ s and an energy of at least 100mJ and a method for generating an ion beam.

FIG. 1a

FIG. 1b

## Description

The present invention is related to an intra-operatory ion therapy system for irradiating a patient with laser-accelerated ions, preferably carbon ions, during or immediately after a surgical intervention.

Prior art

Systems for generating ion beams are commonly known. As the energy required for each particle for therapeutic treatment is comparably high, the systems are very large with respect to the required space and therefore in most cases, especially when carbon ion beams are to be generated, those systems do not fit in common operating rooms. Therefore, usually complete facilities have to be built comprising high energy particle accelerators for generating the required ion beams. These ion beams are then focused on the spots to be treated with radiation.

The required amount of input energy and the required space makes the systems unsuitable for permanent and flexible treatment of patients. Although it is known that all treatments with high energy photons or electrons do not require systems with such space requirements and energy requirements, the therapeutic effect that can be achieved with carbon ion beams is much better than this, which could be achieved with high energy photons, electrons or protons.

## Technical problem

Starting from the prior art, it is an objective of the present invention to provide a system for intra-operatory therapeutic treatment of patients by using ion beams, especially carbon ion beams wherein this system should require less space and should be much more efficient with respect to the input energy compared to the achieved energy deposition in the patient.

## Solution

This problem is solved by the system according to claim 1 and the method for generating ion beams according to claim 10. Advantageous embodiments of the invention are provided in the dependent claims.

The system for performing intra-operatory ion beam therapy, according to the present invention comprises a laser radiation source adapted to emit laser radiation, a housing, being arranged and connected to the laser radiation source, such that laser radiation emitted from the laser radiation source can be coupled into the housing, a laser target onto which the laser radiation emitted from the laser radiation source can be focussed and from which surface an ion beam can be generated, a window portion in the housing adapted to let an incident ion beam pass the window portion to the outside of the housing at minimum energy loss and adapted to seal the housing, and an vacuum system for generating lower pressure in the inner space of the housing, a focussing system for focussing the laser radiation on the laser target, holding means for holding the laser target and a control device for controlling the focussing system and the holding means and for adjusting the propagation direction of the ion beam, wherein the laser radiation source is adapted to generate laser pulses with a duration between $10^{-18}$s and $10^{-12}$s and an energy of at least 100mJ, wherein the system is adapted to be applied in intra-operative ion therapy for irradiating a patient. Such a system allows for generating high energy ion beams and is suitable for being provided in common operating rooms and therefore allows for treating many patients in a more efficient manner.

According to one embodiment, the laser target comprises at least one of a diamond-like carbon foil with a thickness of 10-50nm, graphitic carbon layers on a palladium foil with a thickness of $20\mu$m, aluminium and/or gold foil with carbon contamination, gold-hydrocarbon two-layers foil, polypropylene foil, mylar foil, heated diamond hemispheric shells or other suitable solid or liquid materials that are rich in carbon. Corresponding materials allow for generating high intensity carbon ion beams over a significant time. Thereby, the reliability of the system can be improved.

In a further embodiment, the laser radiation source comprises a chirped pulse amplification system. Chirped pulse amplification system only require a small space and therefore enhance the applicability of the system in operating rooms and further have a greater mean time before failure with respect to optical guiding systems included therein compared to common laser amplification systems.

The system can further comprise a focusing system for focussing the laser radiation on a region of the laser target having a diameter of less than $50\mu$m, preferably less than $20\mu$m, most preferably less than $5\mu$m. Focussing the incident laser radiation on a very small spot is necessary in order to generate the required energy density for generating the plasma and, therefore, the ion beam.

Furthermore, the system may comprise an applicator for directing the ion beam towards an external target, the applicator comprising means for regulating the laser target position and orientation and the laser incidence angle, wherein the applicator comprises a pivotable arm through which the ion beam can be guided. This method of directing the ion beam by adjustment of the target orientation and laser incidence angle is much more efficient than prior art systems based entirely on electromagnetic beam control elements. In addition, the applicator may be adapted to generate electromagnetic fields for a fine adjustment of the guidance of the ion beam towards the external target. With such an applicator that is provided in the form of a pivotable arm, the ion beam can be erected on to the target spot (for example, a tumour in the patient) with high accuracy, thereby improving the treatment results and avoiding unintended damage of healthy tissue.

According to a further embodiment, the system comprises one or more absorbers for absorbing secondary and/or background radiation, the absorber comprising lead and/or tungsten. Such absorbers reduce the amount of dangerous radiation being emitted when the ion beam is generated that could damage other people, thereby improving the security of the system.

In a further embodiment, the system comprises one or more passive absorbers adapted to reduce, by absorption, energy of an incident ion beam. Thereby, the desired energy of the ion beam can be adjusted such that a most preferable treatment result is achieved.

In one embodiment, the system is characterized in that it comprises a laser pointer that is adapted to indicate an impact spot on the external target, wherein a control device is adapted to automatically adjust the propagation direction of the carbon ion beam in accordance with the impact spot.

A method for generating an ion beam, preferably a carbon ion beam can be provided by means of one of the above systems comprising a laser radiation source that generates laser pulses with a duration between $10^{-18}$s and $10^{-12}$s and an energy of at least 100mJ and that generates a lower pressure in the inner space of a housing, wherein the laser radiation is coupled into the housing and focussed onto a laser target in the housing, wherein an ion beam is generated from the laser target by the incident laser radiation, and wherein the propagation direction of the ion beam is controlled by a control device that adjusts the orientation of the laser target with respect to the propagation direction of the laser radiation. Thereby a method for treating for example, cancer patients, is provided that can also be used in common operating rooms thereby improving the applicability of such radiation therapies.

In one embodiment of the method, the ion beam is generated by radiating a laser target comprising at least one of a diamond-like carbon foil with a thickness of 10-50nm, graphitic carbon layers on a palladium foil with a thickness of $20\mu m$, aluminium and/or gold foil with carbon contamination, gold-hydrocarbon two-layers foil, polypropylene foil, mylar foil, heated diamond hemispheric shells or other suitable solid or liquid materials that are rich in carbon with laser radiation emitted from the laser radiation source. Radiating such foils with laser radiation in order to generate ion beams especially carbon ion beams improves the efficiency of such a system and provides a highly effective treatment method.

According to a further embodiment, the method is characterized in that the laser radiation emitted from the laser radiation source is guided by an optical guiding system from the laser radiation source to the laser target. Thereby, the laser radiation can focus very accurately onto the laser target within the housing, thereby generating an already well focused ion beam wherein only a minor amount of scattered radiation is generated.

In accordance with a further embodiment, the laser radiation is focussed on a region of the laser target by a focussing system, wherein the region has a diameter of less than $50\mu m$, preferably less than $20\mu m$, most preferably less than $5\mu m$. By generating a correspondingly focused spot, very high energy ion beams can be generated.

Still further, the ion beam emitted from the laser target is guided by an applicator onto an external target, wherein the ion beam may be guided by means of electromagnetic fields and/or means for regulating the laser target orientation and laser incidence angle such that the ion beam incites on the external target. This allows for focusing the ion beam precisely on the spot which is to be treated, for example, a tumour within the patient's body. Thereby damage to surrounding healthy tissue can be minimized or even avoided.

Moreover, the energy of the ion beam may be reduced from the initial value $E_1$ to a value $E_2$ by passive absorbers, wherein the number of passive absorbers used to reduce the energy of the ion beam depends on the value $E_2$. This allows for generating custom made ion beams that are specially adapted for the treatment of each patient taking into account the depth in which the tumours to be treated at the required energy.

In one embodiment, the energy value $E_2$ of the ion beam is calculated based on the penetration depth of ions in a known or estimated distribution of tissue densities inside the external target. This allows for taking into account the specific absorption characteristic of human tissue and fluids like blood. Thereby, the negative influence of the ion beams on healthy tissue can be reduced.

In another embodiment, a patient is treated with the generated ion beam during or right after a surgical intervention. This treatment provides significant advantages over traditional treatments, since it is less invasive and causes only minimal damage to surrounding tissue of the external target.

Further, the invention provides a method for treating a patient with an ion beam, preferably a carbon ion beam, during or immediately after a surgical intervention can be provided by means of one of the above systems comprising a laser radiation source that generates laser pulses with a duration between $10^{-18}$s and $10^{-12}$ s and an energy of at least 100mJ and that generates a lower pressure in the inner space of a housing, wherein the laser radiation is coupled into the housing and focussed onto a laser target in the housing, wherein an ion beam is generated from the laser target by the incident laser radiation, and wherein the propagation direction of the ion beam is controlled by a control device that adjusts the orientation of the laser target with respect to the propagation direction of the laser radiation. Thereby a method for treating for example, cancer patients, is provided that can also be used in common operating rooms thereby improving the applicability of such radiation therapies.

In one embodiment of the method, the ion beam is generated by radiating a laser target comprising at least one of a diamond-like carbon foil with a thickness of 10-50nm, graphitic carbon layers on a palladium foil with a thickness of 20pm, aluminium and/or gold foil with carbon contamination, gold-hydrocarbon two-layers foil, polypropylene foil, mylar foil, heated diamond hemispheric shells or other suitable solid or liquid materials that are rich in carbon with laser radiation emitted from the laser radiation source. Radiating such foils with laser radiation in order to generate ion beams especially carbon ion beams improves the efficiency of such a system and provides a highly effective treatment method.

According to a further embodiment, the method is characterized in that the laser radiation emitted from the laser radiation source is guided by an optical guiding system from the laser radiation source to the laser target. Thereby, the laser radiation can focus very accurately onto the laser target within the housing, thereby generating an already well focused ion beam wherein only a minor amount of scattered radiation is generated.

In accordance with a further embodiment, the laser radiation is focussed on a region of the laser target by a focussing system, wherein the region has a diameter of less than $50\mu m$, preferably less than $20\mu m$, most preferably less than $5\mu m$. By generating a correspondingly focused spot, very high energy ion beams can be generated.

Still further, the ion beam emitted from the laser target is directed by an applicator onto an external target, wherein the applicator comprises means for regulating the laser target position and orientation and the laser incidence angle such that the ion beam impinges on the external target. This method requires much less space and energy than the guidance of the accelerated ion beam only by means of electromagnetic fields. Furthermore, the accelerated ion beam may be fine controlled by means of electromagnetic fields. This allows for focusing the ion beam precisely on the spot which is to be treated, for example, a tumour within the patient's body. Thereby damage to surrounding healthy tissue can be minimized or even avoided.

Moreover, the energy of the ion beam may be reduced from the initial value $E_1$ to a value $E_2$ by passive absorbers, wherein the number of passive absorbers used to reduce the energy of the ion beam depends on the value $E_2$. This allows for generating custom made ion beams that are specially adapted for the treatment of each patient taking into account the depth in which the tumours to be treated at the required energy.

In one embodiment, the energy value $E_2$ of the ion beam is calculated based on the penetration depth of ions in a known or estimated distribution of tissue densities inside the external target. This allows for taking into account the specific absorption characteristic of human tissue and fluids like blood. Thereby, the negative influence of the ion beams on healthy tissue can be reduced.

## Brief description of the Figures

Figure 1 a-b    is a schematic depiction of a system according to one embodiment.

Figure 2 a-c    shows an optical guiding system and an applicator according to one embodiment of the invention.

Figure 3 a-b    shows a schematic depiction of absorbers according to two embodiments.

## Detailed description

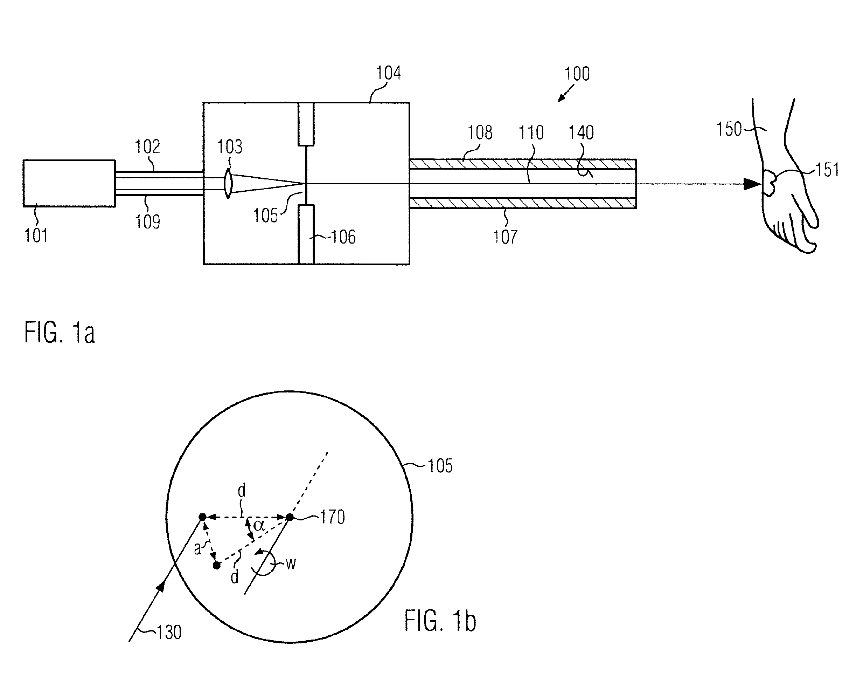

Figure 1 a shows a schematic depiction of a system for generating an ion beam according to one embodiment of the invention. This system 100 comprises a laser radiation source 101. This laser radiation source can be any source as long as it provides sufficient energy beam intensity and pulse duration. For oncologic treatment, for example, carbon ions with energies of about 40 MeV/u are used. This is, compared to other systems like high energy accelerators yielding up to 400 MeV/u, a relatively low energy, but allows for superficial treatment of tumours, for example. Further, employing small ion energies allows for reducing the size of guiding systems and absorbers that absorb high energy particles or radiation. It is, therefore, preferred, that the laser radiation source can generate, in connection with the laser target, carbon ion beams with energies between 20 MeV/u up to 40MeV/u, preferably up to 75 MeV/u. But the laser radiation source in combination with the laser target may not be suitable to provide carbon ion beams with more energy per nucleon. By providing a system that only achieves comparably low energies, the size of the system can be reduced which is a significant advantage compared to large scale accelerators. In order to achieve the intended energy of the accelerated ions, the laser source used should provide lasers pulses having a duration between $10^{-18}$s and $10^{-12}$s wherein each pulse should have an energy of at least 100mJ. Preferably, the pulse repetition rate of the laser pulses ranges from 1 to 1000 Hz.

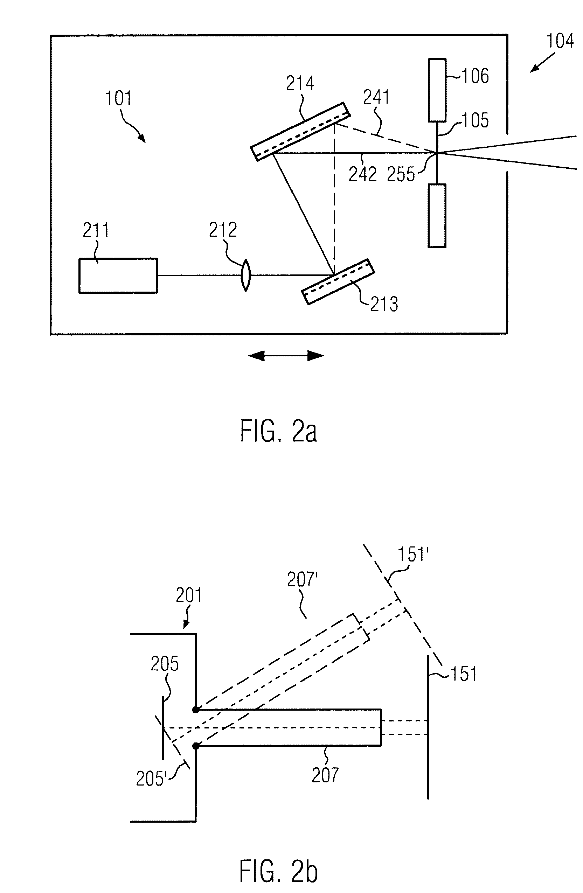

Advantageously, the laser radiation source 101 is a Chirped Pulse Amplification system as will be described with reference to Figure 2a. Independent of the actual realization of the laser radiation source 101, this source is coupled by a focusing system 102 and 103 to a housing 104. Through this focusing system, the emitted laser pulses are focused on a laser target 105. This laser target 105 is situated in the housing 104. The laser target 105 is preferably a foil like a diamond-like carbon foil with a thickness of about 10-15 nm. Other suitable materials are graphitic carbon layers on palladium foil with a thickness of about $20\mu m$, aluminium and/or gold foils with carbon contamination or gold-hydrocarbon two-layer foils or polypropylene or mylar foils or heated diamond hemispheric shells. In principle, all suitable solid or liquid materials may be used, as long as they are rich in carbon and as long as they are suitable laser targets for being radiated with the laser pulses. All these foils have in common that upon radiation with the laser pulses on one side, a plasma is generated, producing high energy carbon ions on the other side of the foil. Although the described system can be used to generate any high energy heavy ion beams, generating carbon ion beams is most preferred as these are used in therapeutic treatments, for example, for cancer patients. If, for example, hydrogen ion beams are to be generated another laser target would have to be used. The provided laser target materials only represent examples. It is possible to use other laser targets as well, as long as they are rich in carbon such that generating a carbon ion beam from the laser target material upon radiation with the laser pulses is possible.

The generated ion beam 110 is then transmitted from the foil 105 to the external target 151 which could be for example cancer cells within a target body 150. The target body 150 shown here represents a target in general, not necessarily a human body, although the invention is intended to be applicable to superficial treatment of cancer. Although the ion beam 110, could be directly emitted from the foil or laser target 105 on to the target body 150, it can be advantageous to provide an applicator 107 that guides the ion beam in a specific manner to the external target 151 in the body 150, whereby the energy spectrum of the ion beam is manipulated in order to fulfil the requirements of the treatment. This applicator will be described later. However, this applicator 107 can generally include a guiding system 108 that guides ion beams 110 to the desired incident spot and further can be used to absorb unwanted radiation. The guiding system 108 may comprise some or all of the later described components, like electromagnetic fields or absorbers.

As the system for generating the ion beam is intended to be also applicable for treating for example cancer patients in common operating rooms, it is preferred that the system fulfils the standard requirements with respect to sterility. Since the system for generating an ion beam is to be used intra-operatively, i.e. during surgery of a patient with a surgically opened patient body, the maintenance of sterile conditions can be advantageous. Therefore, a hull or cover (not shown) can be provided that can be cleaned or disinfected easily. Advantageously, this cover consists of a synthetic material, like a plastic material. In some embodiments, this material itself can have antibacterial properties. Further, the cover may be formed of as few pieces as possible to avoid gaps in which bacteria could grow. Additionally, the cover may be formed edgeless or may have a curved surface such that it is easy to clean. A handle for adjusting the position of the applicator may also be provided in order to ease handling the applicator and may be made of a material comprising antibacterial properties or the handle may be coated accordingly. As the handle will be touched several times during operations, the handle is made, in one embodiment, of a material that has even better antibacterial properties than the remaining cover in order to prevent bacteria growth on the handle that could spread to the rest of the applicator. Also, the control devices for controlling the system may comprise interactive touch displays as these are easier to clean. As the housing 104 and the laser radiation source 101 may be more difficult to disinfect, the applicator may be guided through a separating wall, thereby separating the actual operating room from another room in which the housing, together with the laser radiation source 101, is situated. This can also be advantageous with respect to the cooling, as no sterile air has to be used to cool the laser radiation source 101 and the housing 104. Thereby, the sterility requirements of the applicator (or the part of the applicator that is actually situated in the operating room) can be fulfilled and the necessary cooling can be ensured without risking infections.

With reference to Fig. 1b, it is noted that the laser pulses that impinge on the laser target may have a destructive influence on the laser target. Considering a solid state laser target like a thin foil, radiating the very same point of the foil all the time may lead to complete destruction of this and surrounding parts of the foil because of heating of the incident spot and unhinging of particles of the foil. Therefore, it is preferred that, if the laser target is a foil, the laser target is held by a holding means as shown in Figure 1b. Preferably, this holding means allows for rotating the laser target 105 in a plane perpendicular to the propagation direction of the outgoing ion beam. The laser target 105 may be a disc. In this case, the axis of rotation is preferably in the centre 170 of the disc to avoid disadvantageous torque. The axis of rotation is, in this embodiment, parallel to, but not coinciding with, the propagation direction of the laser pulse 130. This, however, does not need to be the case in all embodiments. The propagation direction of the incident laser pulse 130 and the axis of rotation may include any angle, as the incident angle of the laser pulse on the surface of the laser target has no influence on the actual propagation direction of the outgoing ion beam. Rotating the laser target results in different parts of the laser target being radiated with the laser pulses. Depending on the angular velocity $\omega$ of the laser target and the distance d between the axis of rotation and the propagation direction of the laser pulse, the incident points of two different laser pulses differ from each other in an angular distance $\alpha = \omega t$. The actual distance $a$ corresponding to this angular distance is then given by $a = 2d \cdot \tan\left(\dfrac{\alpha}{2}\right)$. Accordingly, in order to achieve separation of the incident spots of two consecutive laser pulses, the rotation velocity $\omega$ has to be adjusted in accordance with the repetition rate of the laser pulses. In the present invention, this repetition rate ranges from 1 to 1000 Hz, preferably 10 to 1000 Hz.

Hence, as the laser target will extend over a distance of up to 30cm in diameter, rotation velocities of $\omega \approx 0{,}2\text{-}0{,}5\pi s^{-1}$ will be sufficient, as the distance between two incident spots will then be in the order of 5mm, depending on where the actual incident spots are in relation to the centre of the disc, ensuring sufficient separation of the incident points of consecutive laser pulses to avoid undesirable damage to the laser target. It might be even sufficient to let the distance between the incident spots be about 2mm. The corresponding rotation velocities can then be much smaller. It might also be advantageous to provide an automated changing system, that can change the used discs automatically. The already used disc may be guided to the outside of the housing or may be stored in the housing itself until it can be opened after use, as otherwise the low pressure within the housing would be at stake. After releasing the used disc from the holding means, a new disc can be provided to the holding means.

It is noted that it is also possible to provide the laser target (if it is a thin foil) wound on a pair of spools and to unwind it in a direction that crosses the propagation direction of the laser pulse. Thereby, one point of the laser target will always be hit only once by a laser pulse. Regarding the unwinding velocity, corresponding considerations as provided with respect to the rotating disc yield a preferred velocity of about $1ms^{-1}$ in case the repetition rate of the laser pulses is between 1 and 1000 Hz. Further, it is also possible to use droplets that are injected into the housing, such that they come across the propagation direction of the laser pulses. Those droplets, like the already described solid laser targets, must be rich in carbon in order to allow generation of carbon ion beams. If ion beams other than carbon ion beams are to be generated, the laser targets have to be changed accordingly such that they are, for example, rich in hydrogen.

Figure 2a shows an exemplary realization of the laser radiation source 101. Here the laser radiation source is included in the housing 104 as is the foil 105 with the corresponding holding means 106. In this embodiment, the laser radiation source comprises a Chirped Pulse Amplification system. This Chirped Pulse Amplification system comprises a source 211 to deliver a chirped pulse and a compressor. This compressor comprises a first manipulation device 213 which is in this case an optical grating and a second manipulation device which might also be an optical grating 214 wherein these are capable of wavelength dependent manipulating the propagation direction of the incident chirped pulse. Furthermore, the chirped pulse amplification system comprises a focusing device 212 which has a pre-determined focus point 255 that is set to be on the laser target 105. In the arrangement shown in Figure 2(a), the second optical grating 214 is arranged after the first optical grating 213 and both are capable of manipulating the propagation direction of the chirped pulse such that the chirped pulse is transformed after passing the second manipulation device into a pulse wherein different chromatic parts of the pulse are spatially spread but temporarily fully aligned. Here, temporarily fully aligned means that the necessary refocusing of the spatial spread chromatic parts 241 and 242 can be achieved by using non-chromatic devices like mirrors or lenses such that each and every chromatic part reaches the focus point at the same time as the others. This is necessary in order to generate the required energy densities for generating plasma. The used non-chromatic devices for refocusing the spatial spread parts preferably are of a very high quality with respect to their focusing properties in order to ensure that the generated incident spot has the required energy density for generating the plasma.

In this case, the focusing point 255 is a point on the foil 105. Application of a Chirped Pulse Amplification system allows for further miniaturizing the apparatus compared to known particle accelerators or other high energy laser sources. Thereby, the innovative system can be employed even in operating rooms in hospitals. In the European patent application EP 131 624 34.8 examples of the construction of such a chirped pulse amplification system are provided. This document is herewith incorporated by reference. It is noted that in particular content related to the construction of the laser pulse source and the explicit description of its components is incorporated in this application.

By radiating the foil 105 with the generated chirped pulse, a high intensity and high energy laser pulse impinges on the foil 105. Due to the high amount of disposed energy on a very small spot (the focusing device 212 may be designed such that it focuses the laser radiation of the laser pulse on a spot having a diameter of less than $50\mu m$ preferably less than $20\mu m$ and most preferably less than $5\mu m$) a plasma is generated on the opposite side of the foil 105. It should be noted that the cone under which most of the ions are transmitted from the foil is independent from the incident angle of the laser pulse. The ions will be transmitted in a cone with an axis of symmetry that is parallel to the perpendicular of the laser target. Since a foil, i.e. a solid state body, is used, the angle under which the ions with the highest energy are transmitted may differ from the propagation direction of the incident laser pulse. In one embodiment, however, this is used to guide the generated ion beam. Due to the fact that the ions are transmitted approximately parallel to the perpendicular of the laser target, changing the orientation of the laser target allows for changing the propagation direction of the generated ion beams and, therefore, allows for adjusting the incident point of the ion beam on a target body. This method to guide the ion beam by changing the orientation of the laser target requires much less space and energy than systems based on electromagnetic fields acting on the already accelerated ions.

Not all known laser radiation sources are applicable here. First, they have to be adapted to generate the required amount of pulse energy of about 100mJ. Furthermore, the pulse durations of $10^{-18}$s and $10^{-12}$s

must be achieved with the laser radiation source. And, in order to achieve applicability for example in therapeutic application, the repetition rate of the laser pulses must reach 1 up to 1000Hz, such that sufficient energy is disposed for example in tumour cells.

Figure 2b shows one example of the applicator that focuses and redirects the generated ion beam in order to transmit the ions onto the desired part of the target body. As the generated ions only spread in a very small angle, it is not necessary to provide large guiding systems. Indeed, for the present invention, an applicator or handle which is moveably attached to the housing 201, like a pivotable arm, might be sufficient. The applicator 207 is adapted to adjust the position of the laser target when the handle is moved. This means that, when the applicator 207 is moved from its actual orientation to another orientation 207' to hit target body 151' instead of original target body 151, the laser target 205, from which the ions are emitted, is moved accordingly, such that the direction under which the ion beam is transmitted coincides with the new orientation 207'. This can be achieved by mechanically or electrically coupling the applicator 207 to the holding means of the laser target such that a movement of the applicator 207 is translated into a corresponding movement of the laser target 205. In addition to the applicator 207 shown in Figure 2b it can be advantageous to provide electromagnetic fields that focus or redirect the ion beams slightly to ensure that the ion beam impinges on the intended incident point on the target body 151. However, as the energy of the ions is relatively low compared to great accelerators, these systems can be comparably small, if necessary at all. In fact, the provided systems for generating the electromagnetic field may be adapted to only redirect completely ionized carbon ions (i.e. carbon C-12 atoms that were stripped from their electrons completely and, hence, have a charge $q=6e$) with a maximum energy of up 40 MeV/u, preferably up to 75 MeV/u to the external target, but no heavier ions or carbon ions having more energy per nucleon. This is indeed sufficient, as the laser radiation source will not generate plasma comprising carbon ions or heavier ions having higher energies. By providing the corresponding systems, the apparatus can be further miniaturized, as the size of such systems is small compared to the corresponding systems for large scale accelerators.

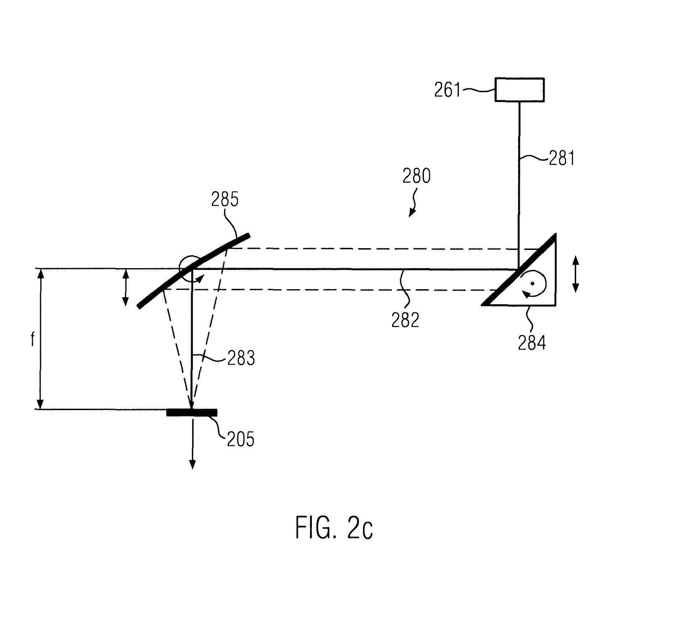

In view of rearranging the laser target 205 upon movement of the applicator 207, rearranging the position of the focal point of the laser pulses may be required as well in order to ensure focussing of the laser pulses on the laser target 205. If this is necessary, it is advantageous to provide means for adjusting the propagation direction and focussing properties of the laser pulses as shown in Figure 2c. Therefore, in addition to the laser radiation source described in Figure 2a, Figure 2c includes some further components.

In the embodiment shown in Figure 2c, an optical guiding system or focussing 280 is arranged between the laser radiation source 261 and the laser target 205. The laser radiation source may be the chirped pulse generator as shown in Figure 2a, or any other appropriate laser radiation source. The optical guiding system 280 includes a first mirror 284 and a parabolic mirror 285, wherein, in this embodiment, the first mirror 284 is placed in propagation direction of the laser pulse before the parabolic mirror 285. In other embodiments, the mirror 284 may be arranged after the parabolic mirror 285 or more than one mirror may be used. The components of the optical guiding system 280 are configured to be moveable. Therefore, the mirror 284, as well as the parabolic mirror 285, may be adapted to rotate around an axis perpendicular to the image plane. Further, the distance between the mirror 284 on the one hand, and the parabolic mirror 285 and the laser target 205 on the other hand, may be variable to adjust the horizontal position of the outgoing ion beam with respect to the external target. Still further, the mirror 284 may be adapted to be moved towards or away from the laser radiation source 261 as indicated by the arrow.

Moving the mirror 284 allows for adjusting the incident point of the laser pulse on the parabolic mirror 285 which is used to focus the laser pulse on the laser target 205 in order to generate the ion beam. If the laser target 205 is moved, for example to follow the movement of the applicator 207, this may result in a shift of the intended incident point of the laser pulse on the laser target 205 in relation to the parabolic mirror 285. This means that, without adjusting the distance f between the parabolic mirror and the laser target 205 as well as the angular orientation of the parabolic mirror 285, the focus point of the laser pulse may no longer be on the laser target 205 after moving the same for example in accordance with the movement shown in Figure 2b. Therefore, the optical guiding system 280 is preferably coupled to the holding means of the laser target 205 such that a movement of the laser target causes a corresponding movement of the optical guiding system 280, such that the focus point of the laser pulse on the laser target 205 remains the same. This is achieved by moving the mirror 284 and the parabolic mirror 285 in accordance with translational and rotational movements of the laser target 205. Thereby, the paths the emitted pulse 281 moves along after passing the mirror 284 (path 282) and the parabolic mirror 285 (path 283) are changed such that the focal point experiences a movement in accordance with the movement of the laser target.

It is noted that the mirror 284 and the parabolic mirror 285 may also be adapted to move independently of the movement of the laser target. Thereby, the position of the incident point of the laser pulse on the laser target 205 can be changed for example such that the trajectory that the incident points of laser pulses move along is, for example, a circle or a curved line. This not only allows for reducing stress on the incident point on the laser target 205 and, therefore, reducing damage that occurs by repeatedly irradiating one spot on the laser target with the laser pulses, but also allows the ion beams to move slightly. This can be advantageous in case it is intended to irradiate, with the ion beams, a larger area for example in a body of a patient. By moving the incident spots of the laser pulses, the corresponding ion beams are generated at different spots and travel through the guiding system 207 on slightly different paths. Hence, the incident points on the target body 151 may differ slightly, allowing for large-area energy deposition if necessary.

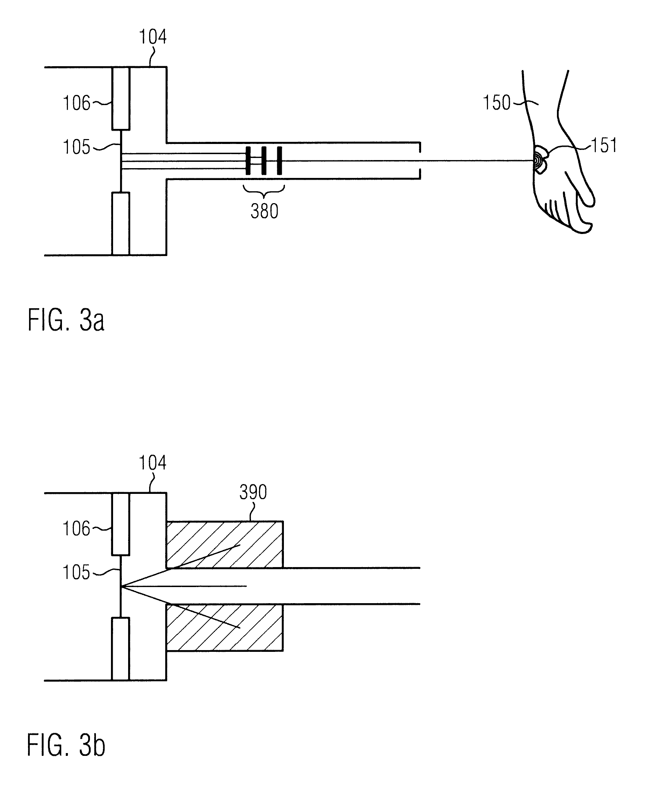

Figure 3a shows one embodiment of passive absorbers that absorb ions reducing the energy of the carbon ion beam due to interaction with the carbon ions. As an example, if the incident laser pulses generate carbon ions with an energy of approximately 40 MeV/u (including a spread of the kinetic energy of the carbon ions), this energy might be too high to be used for treatment of a patient. Therefore, it might be advantageous to first only select those carbon ions having a specific velocity and therefore kinetic energy, for example, by means of the applicator according to Figure 2b and after that, reduce their energy without reducing the amount of carbon ions. This can be achieved by the passive absorbers 380. These absorbers can be made of lead or tungsten or copper or other materials or alloys that are capable of absorbing part of the kinetic energy of the carbon ions. These materials will interact with the carbon ions through ionization processes, wherein the ionized carbon ions cause ionization in the absorbing material, thereby partially loosing their kinetic energy. Thereby, the energy of the carbon ions is reduced but their propagation direction is not changed. Thus, by placing specifically designed absorbers (thickness, density) in the propagation direction of the carbon ion beam, the kinetic energy of the carbon ions can be adjusted as appropriate. In order to achieve this, only one or a plurality of such passive absorbers might be placed in the propagation direction of the carbon ion beam wherein the passive absorbers can be controlled such that they are either in the propagation path of the carbon ions thereby reducing the energy by a specific amount or not. The amount of energy reduction may depend on the necessary penetration depth of the carbon ions in water or water equivalent tissue. Therefore, depending on the position of cancer cells within the patient's body, the required amount of energy reduction of the generated carbon ions can be calculated, since the penetration depth of the carbon ions as a function of their energy is well known.

Furthermore, as the generation of the carbon ions from the foil 105 can only be achieved by generating a high energy plasma at the impinging spot of the laser pulse, secondary radiation and background radiation like gamma radiation is generated. This radiation is, however, in most cases dangerous. Therefore, further absorbers, 390 as shown in Figure 3b can be provided in the area of the generation of the carbon ions such that unintended background or secondary radiation is absorbed and does not reach the outside of the device. Preferably, these absorbers 390 are arranged around the applicator and near the housing and the ion generation spot.

It should be noted that the generation of such high energy plasma requires conditions of near vacuum. Therefore, the housing in which the foil is placed and the applicator which guides the carbon ions to the patient is preferably completely evacuated or has a pressure of less than 1 ten thousandth part of normal pressure. Thus, the plasma can be generated and the spread of the carbon ion beam is reduced and further the carbon ions travels without further collision with other particles through the housing and the applicator. In order to keep the pressure at this value, the complete housing has to be sealed. This provides the difficulty that the carbon ions on the other hand have to leave the housing or the applicator in order to impinge on the external target or the target body. This is achieved by providing a window portion that the carbon ions can pass with minimum energy loss wherein this window still seals the housing. Appropriate materials for such window portions can be Kapton foils having a thickness of a few micrometers. These Kapton foils can resist pressure differences of 1 bar over an area of some square centimetres. As the carbon ion beam is preferably focused on only a very small spot within the human body (usually some millimetres or centimetres) such a small window portion having an area of less than 10 cm$^2$ can be sufficiently large for emitting the necessary carbon ion beams but is still small enough to prevent air from outside the housing to enter the housing thereby maintaining the low pressure.

Furthermore, it might be advantageous to guide the carbon ion beam onto the incident spot on the human body (usually the carbon ions will travel some centimetres through healthy tissues before reaching the cancerous cells) by automated means. This can be achieved by providing a laser pointer that indicates the intended impact spot of the carbon ions wherein a control device automatically adjusts the arrangement of the laser target relative to the laser radiation source and/or that automatically adjusts the orientation of the applicator which influences the propagation direction of the carbon ion beam. Thereby, a highly reliable guiding system for the carbon ions is provided, thereby reducing the likelihood of radiating healthy cells with the carbon ions.

## Claims

1. Intra-operatory ion therapy system for irradiating a patient with laser-accelerated ions, preferably carbon ions, henceforth referred to as "Ion therapy system", comprising a laser radiation source adapted to emit laser radiation, a housing, being arranged and connected to the laser radiation source, such that laser radiation emitted from the laser radiation source can be coupled into the housing, a laser target onto which the laser radiation emitted from the laser radiation source can be focussed and from which surface an ion beam can be generated, a window portion in the housing adapted to let an incident ion beam pass the window portion to the outside of the housing at minimum energy loss and adapted to seal the housing, a vacuum system for generating lower pressure in the inner space of the housing, a focussing system for focussing the laser radiation on the laser target, holding means for holding the laser target and a control device for controlling the focussing system and the holding means and for adjusting the propagation direction of the ion beam such that the ion beam impinges on an external target, wherein the laser radiation source is adapted to generate laser pulses with a duration between $10^{-18}$s and $10^{-12}$ s and an energy of at least 100mJ.

2. Ion therapy system according to claim 1, wherein the laser target comprises at least one of a diamond-like carbon foil with a thickness of 10-50nm, graphitic carbon layers on a palladium foil with a thickness of 20μm, aluminium and/or gold foil with carbon contamination, gold-hydrocarbon two-layers foil, polypropylene foil, mylar foil, heated diamond hemispheric shells or other suitable solid or liquid materials that are rich in carbon.

3. Ion therapy system according to claim 1 or 2, wherein the laser radiation source comprises a chirped pulse amplification system.

4. Ion therapy system according to any of claims 1 to 3, further comprising a focussing system for focussing the laser radiation on a region of the laser target having a diameter of less than 50μm, preferably less than 20μm, most preferably less than 5μm.

5. Ion therapy system according to claim 4, further comprising an optical guiding system for guiding the laser radiation from the laser radiation source to the focussing system.

6. Ion therapy system according to any of claims 1 to 5, further comprising an applicator for guiding the ion beam towards an external target, the applicator comprising means for regulating the laser target position, laser target orientation, and laser incidence angle to direct the ion beam towards the external target, wherein the applicator comprises a pivotable arm through which the ion beam can be guided and wherein the applicator may further be adapted to generate electromagnetic fields to fine adjust the guidance of the ion beam towards the external target.

7. Ion therapy system according to any of claims 1 to 6, further comprising one or more absorbers for absorbing secondary and/or background radiation, the absorber comprising lead and/or tungsten.

8. Ion therapy system according to any of claims 1 to 7, further comprising one or more passive absorbers adapted to reduce, by absorption, energy of an incident ion beam.

9. Ion therapy system according to any of claims 1 to 8, **characterized in that** the system comprises a laser pointer that is adapted to indicate an impact spot on the external target, wherein a control device is adapted to automatically adjust the propagation direction of the carbon ion beam in accordance with the impact spot.

10. Method for generating an ion beam, preferably a carbon ion beam, by means of a system comprising a laser radiation source that generates laser pulses with a duration between $10^{18}$s and $10^{-12}$s and an energy of at least 100mJ and a vacuum system that generates a lower pressure in the inner space of a housing, wherein the laser radiation is coupled into the housing and focussed onto a laser target in the housing, wherein an ion beam is generated from the laser target by the incident laser radiation and wherein the propagation direction of the ion beam is controlled by a control device that adjusts the orientation of the laser target with respect to the propagation direction of the laser radiation such that the emitted ion beam incides on an external target.

11. Method according to claim 10, wherein the ion beam is generated by radiating a laser target comprising at least one of a diamond-like carbon foil with a thickness of 10-50nm, graphitic carbon layers on a palladium foil with a thickness of 20μm, aluminium and/or gold foil with carbon contamination, gold-hydrocarbon two-layers foil, polypropylene foil, mylar foil, heated diamond hemispheric shells or other suitable solid or liquid materials that are rich in carbon with laser radiation emitted from the laser radiation source.

12. Method according to claim 10 or 11, **characterized in that** the laser radiation emitted from the laser radiation source is guided by an optical guiding system from the laser radiation source to the laser target.

13. Method according to any of claims 10 to 12, further **characterized in that** the laser radiation is focussed on a region of the laser target by a focussing system, wherein the region has a diameter of less than 50μm, preferably less than 20μm, most preferably less than 5μm.

14. Method according to any of claims 10 to 13, wherein the ion beam emitted from the laser target is guided by an applicator onto an external target, wherein the ion beam is guided by means of electromagnetic fields and/or means for regulating the laser target orientation and laser incidence angle such that the ion beam incides on the external target.

**15.** Method according to any of claims 10 to 14, wherein the energy of the ion beam is reduced from the initial value $E_1$ to a value $E_2$ by passive absorbers, wherein the number of passive absorbers used to reduce the energy of the ion beam depends on the value $E_2$. I

**16.** Method according to claim 15, wherein the energy value $E_2$ of the ion beam is calculated based on the penetration depth of ions in a known or estimated distribution of tissue densities inside the external target.

**17.** Method according to any of claims 10 to 16, **characterized in that** a patient is treated with the generated ion beam during or right after a surgical intervention.

FIG. 1a

FIG. 1b

EP 2 887 379 A1

FIG. 2a

FIG. 2b

FIG. 2c

EP 2 887 379 A1

FIG. 3a

FIG. 3b

15

Europäisches
Patentamt

European
Patent Office

Office européen
des brevets

## EUROPEAN SEARCH REPORT

Application Number

EP 13 38 0060

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/090194 A1 (TAJIMA TOSHIKI [US]) 11 July 2002 (2002-07-11) * paragraphs [0016] - [0027]; figures 1A-1C * | 1-17 | INV. H01J27/24 ADD. H01J33/00 A61N5/10 |
| A | A-C WRA ET AL: "In vitro irradiation station for broad beam radiobiological experiments", NUCLEAR INSTRUMENTS & METHODS IN PHYSICS RESEARCH, SECTION - B:BEAM INTERACTIONS WITH MATERIALS AND ATOMS, vol. 269, no. 24, 28 April 2011 (2011-04-28), pages 3120-3124, XP028118280, ISSN: 0168-583X, DOI: 10.1016/J.NIMB.2011.04.104 [retrieved on 2011-04-28] * page 3121, left-hand column; figure 1 * | 1-17 | |
| A | US 2006/145088 A1 (MA CHANG-MING C [US] MA CHANG-MING CHARLIE [US]) 6 July 2006 (2006-07-06) * the whole document * | 1-16 | |
| A | R. MEESAT ET AL: "Cancer radiotherapy based on femtosecond IR laser-beam filamentation yielding ultra-high dose rates and zero entrance dose", PROCEEDINGS OF THE NATIONAL ACADEMY OF SCIENCES, vol. 109, no. 38, 18 September 2012 (2012-09-18), pages E2508-E2513, XP055127181, ISSN: 0027-8424, DOI: 10.1073/pnas.1116286109 * the whole document * | 1-17 | TECHNICAL FIELDS SEARCHED (IPC) H01J A61N |

The present search report has been drawn up for all claims

| Place of search | Date of completion of the search | Examiner |
|---|---|---|
| The Hague | 8 July 2014 | Loiseleur, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 38 0060

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2014

| Patent document cited in search report | Publication date | Patent family member(s) | Publication date |
|---|---|---|---|
| US 2002090194 A1 | 11-07-2002 | US 2002090194 A1<br>US 2005167610 A1 | 11-07-2002<br>04-08-2005 |
| US 2006145088 A1 | 06-07-2006 | AU 2004246641 A1<br>CA 2525777 A1<br>CN 101006541 A<br>EP 1629508 A2<br>JP 2007525249 A<br>US 2006145088 A1<br>WO 2004109717 A2 | 16-12-2004<br>16-12-2004<br>25-07-2007<br>01-03-2006<br>06-09-2007<br>06-07-2006<br>16-12-2004 |

EPO FORM P0459

For more details about this annex : see Official Journal of the European Patent Office, No. 12/82